# EUROPEAN PATENT APPLICATION

(11) **EP 3 384 987 A2**
(43) Date of publication of application: **10.10.2018**
(21) Application number: 18165019.3
(22) Date of filing: 29.03.2018
(51) Int. Cl.: B01L 3/00, B29C 51/10, B29C 51/12, G01N 27/28, G01N 27/403

(54) **VESSEL FOR PERFORMING ELECTROCHEMICAL MEASUREMENTS AND METHOD FOR MANUFACTURING SUCH VESSEL**

(30) Priority: 03.04.2017 EP 17164486
(71) Applicant: CSEM Centre Suisse d'Electronique et de Microtechnique SA - Recherche et Développement, 2002 Neuchâtel (CH)
(72) Inventor: Glaser, Nicolas, 4056 Basel (CH); Kristiansen, Magnus, 8708 Männedorf (CH); Wollmann, Sebastian, 5415 Nussbaumen (CH)
(74) Representative: P&TS SA (AG, Ltd.)

(57) **Abstract**

The present invention concerns a vessel (1) for performing electrochemical measurements comprising at least a well (10) for receiving a solution, the well (10) having an upper portion (13), a bottom portion (12) and a side wall portion (11) extending between the upper portion (13) and the bottom portion (12), wherein the upper portion (13), the bottom portion (12) and the side wall portion (11) are formed integral, and wherein at least one of the well comprises an electrode (20) located at least partially on the side wall portion (11). The invention further concerns a method for manufacturing the vessel (1), comprising: providing a substantially flat substrate (30); printing the electrode (20) on a surface of the substrate (30); and thermoforming the substrate (30) with the printed electrode (20) in a mold (40) such that the electrode (20) is located at least partially on the side wall portion (11).

## Description

### Field

The present invention concerns a vessel for performing electrochemical measurements and a method for manufacturing such vessel.

### Description of related art

Electrochemical analysis, cell analysis, proteomics and other analytes by detection of various local or internal changes in pH (acidification), ionic strength or redox potential is a promising and attractive method of instrument analysis. Microtiter plates (or well-plates) comprise electrodes can be advantageously used for such measurements among others.

For example, a potentiometric method of electrochemical analysis can be used using an electrochemical sensing electrode comprising a metallic potentiometric electrode coated with a layer of electroconductive polymer containing immobilised bioreceptor molecules which bind specifically to the analyte under test.

Known well-plates comprise electrodes located on the bottom of the wells. The electrodes are typically made on the flat substrate constituting the bottom of the wells. US2015125942 describes a culture plate having a lid for guiding placement of cells and materials in each individual culture well of a culture plate. The lid may provide for coupling to an electrophysiology culture plate comprising a biosensor plate and a biologic culture plate, where the biosensor plate underlies and is coupled to the culture well plate such that each biosensor is operatively coupled to one culture well of the plurality of culture wells.

Known well-plates on the market are typically made of two pieces, electrodes on a bottom plate that is either over molded or glued to the well or vessel.

### Summary

The present disclosure concerns a vessel for performing electrochemical measurements comprising at least a well for receiving a solution, the well having an upper portion, a bottom portion and a side wall portion extending between the upper portion and the bottom portion, wherein the upper portion, the bottom portion and the side wall portion are formed integral, and wherein at least one of the well comprises an electrode on the side wall portion.

The present disclosure further concerns a method for molding functional, printed electrodes into a 3D-shape such as a well in which an electrochemical measurement can be performed.

In particular, it is disclosed a method for manufacturing the vessel, comprising: providing a substantially flat substrate; printing at least one electrode on a surface of the substrate; and thermoforming the substrate with the printed electrode in a mold such that the at least one electrode is located at least partially on the side wall portion,

In a preferred embodiment, the placement of the at least one electrode does not hamper the transparency of a (transparent) well since the electrodes is located on the side of the well but not at its bottom portion.

At least a functionalized electrode, allows the vessel to be used for various measurements such as pH, ion or enzymatic detection.

Alternatively to a measurement function or in combination with it, at least one electrode can be used for electrical stimulation of the solution or of cells located in the solutions or on the surface of the well.

The method disclosed herein allows for manufacturing a vessel that is made in one piece. The disclosed manufacturing method also has high-throughput, compared to possible alternative manufacturing methods such as 3D printing techniques including aerosol jet printing.

### Brief Description of the Drawings

The invention will be better understood with the aid of the description of an embodiment given by way of example and illustrated by the figures, in which:
Fig. 1 represents schematically a vessel comprising an electrode for performing electrochemical measurements, according to an embodiment;
Fig. 2 shows a top view of a multiwell plate comprising a plurality of the vessel, according to an embodiment;
Fig. 3 shows a bottom view of a multiwell plate comprising a plurality of the vessel, according to an embodiment;
Fig. 4 illustrates a top view of a multiwell plate comprising a plurality of wells, according to another embodiment;
Figs. 5a to 5c illustrate a method for manufacturing the vessel, according to an embodiment;
Figs. 6a to 6c illustrate a method for manufacturing the vessel, according to another embodiment;
Fig. 7 illustrates the positioning of the electrode on a substrate, according to an embodiment; and
Fig. 8 shows a method for manufacturing the vessel, according to yet another embodiment.

### Detailed description of possible embodiments

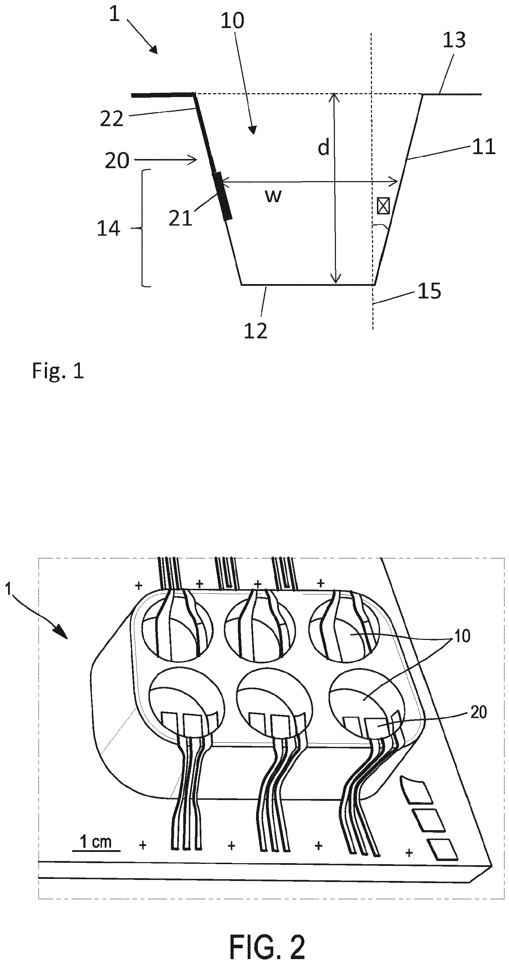

A vessel 1 for performing electrochemical measurements is represented schematically in Fig. 1, according to an embodiment. The vessel 1 comprises a well 10 for receiving a solution. The well 10 comprises an upper portion 13, a bottom portion 12 and a side wall portion 11, extending between the upper portion 13 and the bottom portion 12. The well comprises an electrode 20 on the side wall portion 11. The upper portion 13, the bottom portion 12 and the side wall portion 11 are formed integral, i.e., are made from one piece.

The electrode 20 can be used to measure a specific analyte in a solution or to apply a stimuli such as an electric field to the solution of interest. Electrochemical measurement are particularly interesting for the continuous monitoring of cell culture media. Electroporation or impedance analysis are further applications that are frequently done on biological or chemical specimens

The electrode 20 can comprise an active area 21 that is destined to be in contact with the solution when received in the well 10. The electrode 20 can also comprise a non-active area 22 that is insulated from said solution.

In a possible embodiment, the active area 21 extends in a predetermined portion 14 of the side wall portion 11, between the upper portion 13 down to the bottom portion 12. The non-active area 22 can correspond to electrical wires, insulated from said solution, to be connected to a measurement or to an electrical stimulation apparatus, eventually via a multiplexing electronic circuit. In the example of Fig. 1, the non-active area 22 extends along the side wall portion 11 and the upper portion 13.

The predetermined portion 14 can be in lower half of the well 10. Alternatively, the predetermined portion 14 can be in lower third of the well 10. More generally, the predetermined portion 14 can be in a region of the well 10 that is destined to be completely covered by the solution to be received in the well 10.

The active area 21 of the electrode 20 is thus in contact with the solution and can react with it, for example via an electrochemical reaction.

In an embodiment, the active area 21 can be functionalized such that it can be used for measuring pH, for ion detection such as K⁺, NA⁺, Cl⁻, NH₄⁺, PO₄³⁻, or any other ion and salt detection, for enzymatic detection, for glucose, lactate, glutamate or other nutrient detection, or for electrochemical immunoassay for protein or antigen detection.

The vessel can comprise a well 10 having a straight side wall portion 11, i.e., substantially perpendicular to the upper portion 13 (represented by the dotted line 15 in Fig. 1). Alternatively, the wall portion 11 can make an angle θ relative to a straight wall portion. The angle θ can be smaller than 45°, preferably smaller than 20° and even more preferably smaller than 10°. In general, real geometries manufactured with high throughput methods will exhibit a rounded shape between the upper portion 13 and the side wall portion 11 of the vessel. Similarly, the transition between the bottom portion 11 and the side wall portion 11 of the vessel can have a rounded shape.

The vessel can comprise a well 10 having a ratio of the depth d over the width w of the well 10 is between 1 and 5, or between 2 and 3 or between 1 and 1.5 or between 0.5 and 1. In the case where the wall portion 11 is sloped (non-zero angle θ), the width w can be measured at mi-depth.

The bottom portion 12 can be substantially flat as shown in Fig.1, or can have any other suitable shape. For example, the bottom portion 12 can have a concave shape.

It is understood that the well 10 can comprise more than one electrode 20 being located at least partially on the side wall portion 11.

Fig. 2 shows an example where the vessel 1, viewed form top, comprises a plurality of wells 10, each comprising three electrodes 20. Here, the vessel 1 can be a multiwell plate, for example having a plurality of wells 10. The wells 10 can have a volume between 0.05 to 20 milliliters, and typically between 6 to 96 wells. Or substantially larger vessels up to 500 ml.

The transparent thermoformed vessel 1 is located on a molding device 41 used in thermoforming.

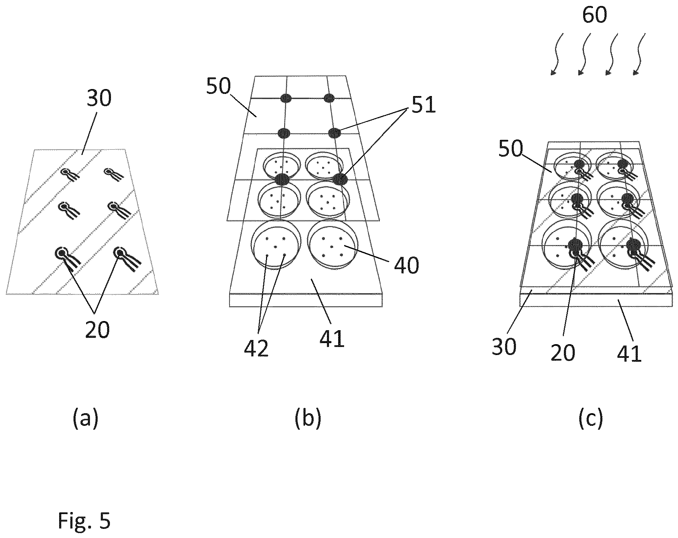

Fig. 5a represents a drawing of a planar foil 30 with six sets of electrodes 20. Each set is designated to be formed into one vessel. Each set of electrodes 20 is constituted of the number of electrodes needed for a desired analysis method. Single analyte measurement require two to four electrodes, whereas multi-analyte measurements may be done with a multitude of differently functionalized electrodes. The electrode 20 is placed at least partially on the side wall portion 11. In particular, the active area 21 of the electrode 20 is located on the side wall portion 11 and the non-active area 22 is running from the active area 21, on the side wall portion 11 and the upper portion 13, out of the well 10 for connection with a measurement apparatus (not shown). A vessel containing a large number of wells 10, such as a well-plate (or multiwell plate) can lead to a large number of electrodes to be contacted electrically to a measurement or to an electrical stimulation apparatus. In can be preferable to use common electrical contact lines or bus-lines in combination with an electrical multiplexing scheme such as an electrical switching matrix. This allows for limiting the number of electrical contacts to be made to the vessel and for limiting the number of electrical measurement and/or stimulation apparatus input/outputs. Such multiplexing is typically based on time division multiplexing.

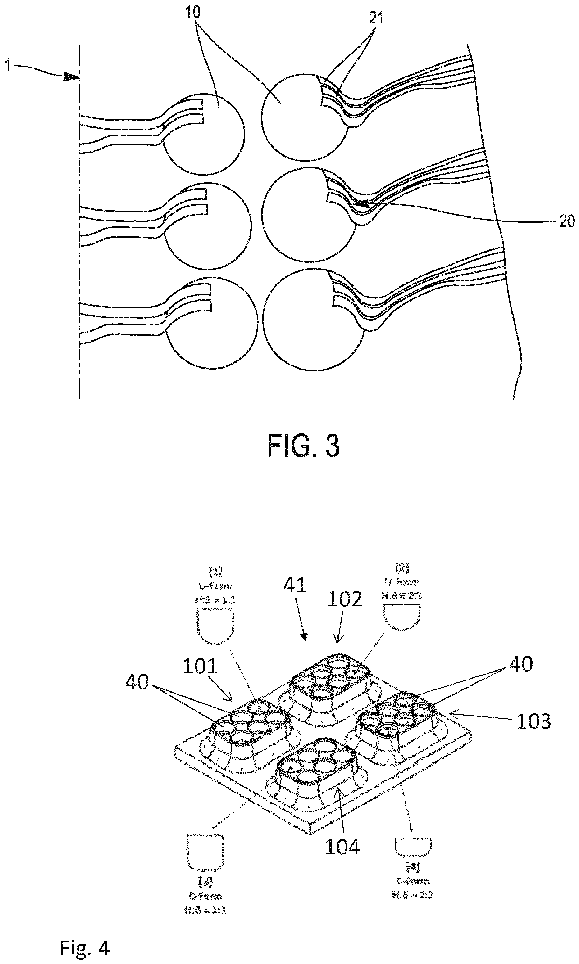

Fig. 3 shows another example of a multiwell plate 1, viewed form bottom, wherein the vessel 1 are made in a transparent plastic. The arrangement of the electrode 20 is the same as the one shown in Fig. 2 however the bottom portion 12 of the wells are concave and not flat as in Fig. 2. The multiwell plate 1 of Fig. 3 shows that the arrangement of the active area 21 of the electrode 20 on the side wall portion 11 can enable for the bottom portion 12 to be transparent (the electrode 20 does not hamper the transparency of the bottom portion 12).

Alternatively, the design of the at least one electrode can contain an active area 21 located at least partially at the bottom portion of the well. This can as an example improve the interaction between the at least active area 21 of an electrode and the well media. This can be of critical importance for cell culture vessels performing cell electro-stimulation or cell electro-impedance characterization.

According to an embodiment, a method for manufacturing the vessel 1 comprises:
providing a substantially flat substrate 30;
printing the at least one electrode 20 on a surface of the substrate 30;
thermoforming the substrate 30 with the printed electrode 20 in a mold 40 such that the electrode 20 is located at least partially on the side wall portion 11.

In an embodiment, the thermoforming step comprises heating the substrate 30 to a temperature that allows the substrate 30 to be stretched into or onto the mold 40, and stretching the substrate 30 to a specific shape in the mold 40 to form the well 10.

Fig. 4 illustrates a top view of a molding device 41 comprising four multiwell molding plates 101, 102, 103, 104, each comprising a plurality of well molds 40. In particular, the molding device 41 comprises a first multiwell molding plate 101 containing well molds 40 having a 1:1 aspect ratio and a concave-shaped bottom portion, a second multiwell molding plate 102 containing well molds 40 having a 2:3 aspect ratio and a concave-shaped bottom portion, a third multiwell molding plate 103 of well molds 40 having a 1:2 aspect ratio and a flat bottom portion with rounded edges, and a fourth multiwell molding plate 104 of well molds 40 having a 1:1 aspect ratio and a flat bottom portion with rounded edges.

The resulting vessel 1 is a multiwell plate (not shown) comprising a first multiwell plate, conform to the multiwell molding plate 101, containing wells 10 having a 1:1 aspect ratio and a concave-shaped bottom portion 12, a second multiwell plate, conform to the second multiwell plate 102, containing wells 10 having a 2:3 aspect ratio and a concave-shaped bottom portion 12, a third multiwell plate, conform to the third multiwell molding plate 103, containing wells 10 having a 1:2 aspect ratio and a flat bottom portion 12 with rounded edges, and a fourth multiwell plate, conform to the fourth multiwell molding plate 104, containing wells 10 having a 1:1 aspect ratio and a flat bottom portion 12 with rounded edges.

Fig. 5a represents a substrate 30 onto which several electrodes 20 have been printed. The substrate 30 can be a foil made of a material and having a thickness adapted for thermoforming. For example, the substrate 30 can comprise a transparent or opaque thermoformable foil, such as polymethylmethacrylate (PMMA), cyclic olefin copolymer (COC), cyclo olefin polymers (COP), polycarbonate (PC), polystyrene (PS), polyethylene terephthalate (PET), polypropylene (PP), polyvinyl chloride (PVC), acrylonitrile butadiene styrene (ABS), polyethylenimine (PEI), polyamide (PI) and others suitable materials.

Printing the electrode 20 can comprise printing a conductive metal ink on a surface of the substrate 30. Printing the electrode 20 can also comprise printing a conductive paste or a conductive carbon-based ink or paste, or printing a poly(3,4-ethylenedioxythiophene) polystyrene sulfonate (PEDOT:PSS) or printing any other organic conductive inks or pastes.

The printing method can comprise screen printing, gravure printing, off-set printing, inkjet-printing, aerosol-jet printing, 3D printing, or any other suitable printing method.

Thermoforming the (substantially flat) substrate 30 can comprise putting the substrate 30 on a molding device 41 (see Fig. 5c). Here, the molding device 41 is a plate comprising a plurality of molds 40. The substrate is heated and is then sucked into the molds 40 by means of vacuum suction. The vacuum suction can be provided through holes 42 provided in the bottom of the molds 40. Heating can be performed by using an infra-red heating source 60 or a hot air heating source 60.

The thermoforming step can be helped with a stamping press (not represented in the figures) having a shape conform with the one of the molds 40. In this case, the molding device and/or the stamping press can be heated.

Preferably, the substrate 30 should be configured for stretching (plastic deformation) below 500% and more preferably below 200% in both directions on areas where electrodes are located. This can be influenced and designed with the molding device geometry or by a stamping press geometry and its heating. Local stretching of the substrate 30 can as well be influenced by using a mask 50 (see Figs. 5b and 5c) adapted for shielding at least a portion of the at least printed electrode, protecting predetermined areas of the substrate 30 from the heating source 60. Local stretching of the substrate 30 can also be influenced by local variation of the substrate thickness or substrate material. As an example, areas where electrode are located can contain an additional polymeric material having a higher stiffness or a higher glass-transition temperature. Local stretching of the substrate 30 can also be adapted locally, for example is the area where the electrode 20 is printed and/or areas of critical strain.

In an embodiment, the method for manufacturing can further comprise a step of functionalizing the electrode 20. In particular, step of functionalizing the electrode 20 comprises functionalizing the active area 21 of the electrode 20.

The functionalization can comprise applying a silver chloride ink or paste. In addition, solution processed chemical agents can be added on top of any of the printed electrodes. The functionalization can comprise protein or antigen used in immunoassays or other biological molecules and compounds. The functionalization can compromise solution deposited membranes, solid state electrolytes or other electrochemical agents.

The method of functionalization can comprise any printing technique, drop casting, physical vapor deposition or electrodeposition.

The step of printing the electrode 20 and functionalizing the electrode 20 can be done on a roll to roll machine or sheet to sheet machine or roll to sheet machine. Alternatively, the functionalization or part of the functionalization can be done by a user, after the manufacturing of the vessel 1 is completed.

In an embodiment, the step of functionalizing is performed prior to thermoforming. In that case, the thermoforming step is preferably performed by using a mask 50 (see Figs. 5b and 5c) adapted for shielding the active area 21 from the heat produced by the heating source 60. In particular, the shielding provided by the mask 50 is advantageous for protecting the delicate functionalization (e.g. biological enzyme) of the active area 21 from the heat. In the example of Figs. 5b and 5c, the mask 50 comprises a pattern of dots 51 opaque to the heat. The mask 50 is inserted between the heating source 60 and the substrate 30 in order to protect the active portion 21 of the electrodes 20 from the heat.

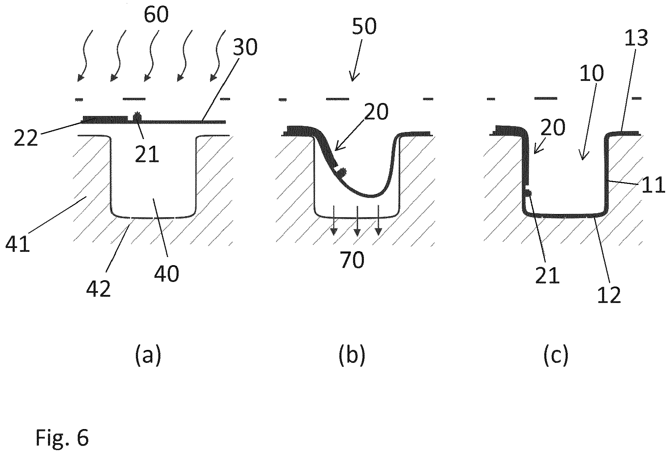

Fig. 6 represents a cross-section view showing the formation of a single well 10 using the method of the invention. In Fig. 6a, the substrate 30 is put over a mold 40 and a heating source 60 heats the substrate 30. The substantially planar substrate 30 comprised the printed electrode 20 containing an active area 21 and a non-active area 22. Once the substrate 30 has been heated to a suitable temperature, it is deformed into the mold 40 by the action of vacuum suction 70 at le bottom of the mold 40 (see Fig. 6b). The deformation step takes place until the substrate 30 is completely deformed conform with the shape of the mold 40. The completely deformed substrate 30 then forms the well 10 comprising the upper portion 13, the bottom portion 12 and the side wall portion 11. Due to the placement of the electrode 20 printed on the substrate 30, the electrode 20 is located at least partially of the side wall portion 11 once the substrate 30 is completely deformed. The heating is in general stopped and a short cooling down time is used before demolding the vessel 1.

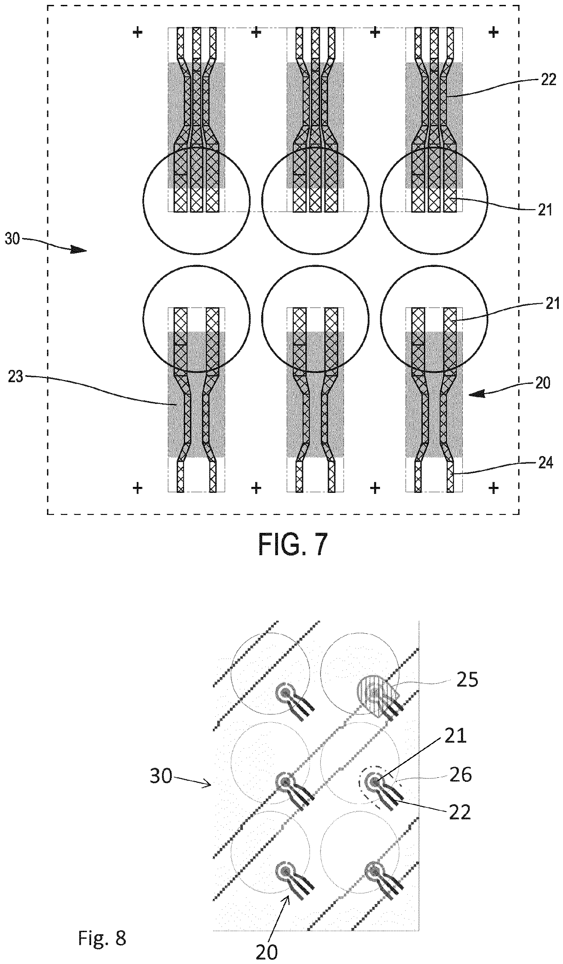

Fig. 7 illustrates an example of positioning the printed electrodes 20 on the substrate 30 such that the active area 21 of the electrode 20 is properly located on the side wall portion 11 after the substrate 30 is thermoformed in the mold 40. In this example, six sets of electrodes 20 are equidistantly printed (three sets of three electrodes 20 and three sets of two electrodes 20, head to head) on the substrate 30 to form a six-well vessel 1. The electrode 20 comprises a non-active area 22 covered with an insulator 23 and an active area 21 located on the side wall portion 11 and destined to react with a solution received in the well 10. The electrode 20 also comprises a non-insulated portion 24 destined to be used as electrical connectors for a measurement and/or stimulation apparatus (not shown).

The step of thermoforming the substrate 30 can be followed by a step of cutting the thermoformed substrate 30 to a final format. This cutting operation is optional and the final format can be obtained directly after the thermoforming step. Alternatively, the step of cutting can be performed before the thermoforming step.

It is understood that the present invention is not limited to the exemplary embodiments described above and other examples of implementations are also possible within the scope of the patent claims.

For example, in an embodiment shown in Fig. 8, the manufacturing method can comprise a step of cutting the substrate 30 or one of its layer, containing the printed electrodes 20 to provide cut portions 25. For example, a partially cut portion 25 can comprise an area comprising the electrode 20. An example of a cutting line is indicated by the numeral 26 around a printed electrode 20 in Fig. 8. For example, the step of providing the cut portions 25 can be performed between the step of printing the electrode 20 and the one of thermoforming the substrate 30.

During thermoforming, the substrate 30 with the cut portions 25 is put on the molding device 41, the substrate 30 is heated and the cut portions 25 are folded in the mold 40 such that the electrode 25 is located at least partially on the side wall portion 11 of the well 10. Folding the cut portions 25 can be performed by using a stamping press having a shape conform with the one of the mold 40. The vacuum suction may not be needed here.

The folding of the cut portions 25 does not necessarily require any stretching of the substrate 30 (and/or the cut portions 25).

The thickness of the substrate 30 can be locally varied (for example increased) in order to stiffen the substrate 30 where needed.

The step of thermoforming the substrate 30 can be preceded by a step of thermoforming a first substrate (not shown), possibly not comprising electrode 20. The first substrate is heating to a temperature that allows the first substrate to be stretched into or onto the mold 40, and stretching the first substrate to a specific shape in the mold 40 to form the well 10. The cut portions 25 of the substrate 30 is then folded on the side wall portion 11 formed by the first substrate. Alternatively, the substrate can contain at least two layers. At least an uncut layer of the substrate will be molded in the well shape and hold the solution, while the cut layer(s) will be molded with minimal stretching of the electrodes. In particular the cut portion 25 can experience a bending during the thermoforming process and not a two-dimensional stretching

Alternatively, the substrate 30 comprising the cut portions 25 can be folded in an existing well 10 (for example a commercially available well, of a vessel, multiwell plate, etc., in order to add the electrode 20 on the side wall portion 11 of the well 10. For example, the substrate 30 comprising the cut portions 25 can be folded on a culture flask, a disposable bioreactor flask, a petri dish, or any other suitable measurement vessel.

### Reference numeral used in the figures

- 1: multiwell plate
- 10: well
- 101: first group
- 102: second group
- 103: third group
- 104: fourth group
- 11: side wall portion
- 12: bottom portion
- 13: upper portion
- 14: predetermined portion
- 15: straight wall portion
- 20: electrode
- 21: active area
- 22: non-active area
- 23: insulator
- 24: non-insulated portion
- 25: cut portion
- 26: cutting line
- 30: substrate
- 40: mold
- 41: molding device
- 42: through holes
- 50: mask
- 51: pattern
- 60: heat source
- 70: vacuum
- d: depth
- w: width

## Claims

1. Vessel (1) for performing electrochemical measurements comprising at least a well (10) for receiving a solution, the well (10) having an upper portion (13), a bottom portion (12) and a side wall portion (11) extending between the upper portion (13) and the bottom portion (12), wherein the upper portion (13), the bottom portion (12) and the side wall portion (11) are formed integral, and wherein at least one of the well comprises an electrode (20) at least partially on the side wall portion (11).

2. The vessel (1) according to claim 1,
wherein the electrode (20) comprises an active area (21) that is destined to be in contact with the solution when received in the well (10) and a non-active area (22) that is insulated from said solution.

3. The vessel (1) according to claim 2,
wherein said active area (21) extends in a predetermined portion (14) of the side wall portion (11), between the upper portion (13) down to the bottom portion (12).

4. The vessel (1) according to claim 3,
wherein said predetermined portion (14) is in lower half of the well (10) or is in lower third of the well (10).

5. The vessel (1) according to any one of claims 1 to 4,
wherein the side wall portion (11) makes an angle (θ) being smaller than 45°, preferably smaller than 20° and even more preferably smaller than 10°.

6. The vessel (1) according to any one of claims 1 to 5,
wherein the ratio depth over width of the well (10) is between 1 and 5, or between 2 and 3 or between 1 and 1.5 or between 0.5 and 1.

7. The vessel (1) according to any one of claims 1 to 6,
wherein the bottom portion (12) is substantially flat or has concave shape.

8. The vessel (1) according to any one of claims 2 to 7,
wherein the non-active area (22) extend between the active area (21) and the upper portion (13).

9. Multiwell plate comprising a plurality of the well (1) according to any one of claims 1 to 8.

10. Method for manufacturing the vessel (1) according to any one of claims 1 to 8, comprising:
providing a substantially flat substrate (30);
printing the electrode (20) on a surface of the substrate (30); and
thermoforming the substrate (30) with the printed electrode (20) in a mold (40) such that the electrode (20) is located at least partially on the side wall portion (11).

11. The method according to claim 10,
wherein thermoforming the substrate (30) comprises vacuum forming.

12. The method according to claim 10 or 11,
further comprising a step of cutting the thermoformed substrate (30).

13. The method according to any one of claims 10 to 12,
further comprising a step of functionalizing the active area (21) of the electrode (20).

14. The method according to claim 13,
wherein the step of functionalizing is performed prior to thermo-forming and wherein the thermo-forming step is performed by using a mask (50) adapted for shielding the active area (21) from the heat.

15. The method according to any one of claims 10 to 14,
wherein printing the electrode (20) comprises a step of positioning the electrode (20) on the substrate (30) such that the electrode (20) is located at least partially on the side wall portion (11) after the step of thermo-forming.

16. The method according to claim 10,
comprising a step of cutting at least one layer of the substrate (30) containing the printed electrode (20) to provide a cut portion (25); and wherein during thermoforming, the substrate (30) with the cut portions (25) is heated and the cut portion (25) is folded in the mold (40) such that the electrode (25) is located at least partially on the side wall portion (11) of the well (10).
